# EUROPEAN PATENT APPLICATION

(11) **EP 3 147 361 A1**
(43) Date of publication of application: **29.03.2017**
(21) Application number: 15795622.8
(22) Date of filing: 21.05.2015
(51) Int. Cl.: C12N 15/09, A01K 67/027, C07K 19/00, C12N 1/15, C12N 1/19, C12N 1/21, C12N 5/10, C12Q 1/02, G01N 33/68

(54) **FUSION PROTEIN SUITABLE FOR MEASUREMENT OF AUTOPHAGY, NUCLEIC ACID ENCODING SAID FUSION PROTEIN, AND USE OF THESE**

(30) Priority: 21.05.2014 JP 2014105319
(71) Applicant: The University of Tokyo, Tokyo 113-8654 (JP)
(72) Inventor: MIZUSHIMA, Noboru, Tokyo 113-8654 (JP)
(74) Representative: Graf von Stosch, Andreas
(86) International application number: PCT/JP2015/064568
(87) International publication number: WO 2015/178444

(57) **Abstract**

A fusion protein including a LC3 protein which includes a first tag and a protein which includes a second tag.

SEQUENCE LISTING

<110>  The University of Tokyo

<120>  FUSION PROTEIN SUITABLE FOR MEASUREMENT OF AUTOPHAGY, NUCLEIC
       ACID ENCODING THE FUSION PROTEIN, AND USE THEREOF

<130>  N-ST007-15P

<150>  JP 2014-105319
<151>  2014-05-21

<160>  2

<170>  PatentIn version 3.5

<210>  1
<211>  2199
<212>  DNA
<213>  Artificial Sequence

<220>
<223>  Artificial Sequence

<400>  1
atggtgagca agggcgagga gctgttcacc ggggtggtgc ccatcctggt cgagctggac      60

ggcgacgtaa acggccacaa gttcagcgtg tccggcgagg gcgagggcga tgccacctac     120

ggcaagctga ccctgaagtt catctgcacc accggcaagc tgcccgtgcc ctggcccacc     180

ctcgtgacca ccctgaccta cggcgtgcag tgcttcagcc gctaccccga ccacatgaag     240

cagcacgact tcttcaagtc cgccatgccc gaaggctacg tccaggagcg caccatcttc     300

ttcaaggacg acggcaacta caagacccgc gccgaggtga agttcgaggg cgacaccctg     360

gtgaaccgca tcgagctgaa gggcatcgac ttcaaggagg acggcaacat cctggggcac     420

aagctggagt acaactacaa cagccacaac gtctatatca tggccgacaa gcagaagaac     480

ggcatcaagg tgaacttcaa gatccgccac aacatcgagg acggcagcgt gcagctcgcc     540

gaccactacc agcagaacac ccccatcggc gacggccccg tgctgctgcc cgacaaccac     600

tacctgagca cccagtccgc cctgagcaaa gaccccaacg agaagcgcga tcacatggtc     660

ctgctggagt tcgtgaccgc cgccgggatc actctcggca tggacgagct gtacaagggt     720

ggcggtggct cgggcggtgg tgggtcgggt ggcggcggat ctatcgatga attgaattcc     780

atgccgtccg agaagacctt caaacagcgc cggagcttcg aacaaagagt ggaagatgtc     840

cggctcatcc gggagcagca ccccaccaag atcccagtga ttatagagcg atacaagggt     900

gagaagcagc tgcccgtcct ggacaagacc aagttccttg tacctgatca cgtgaatatg     960

agcgaactca tcaagataat tagaaggcgc ctgcagctca atgctaacca agccttcttc    1020

ctcctggtga atgggcacag catggtgagt gtgtccacac ccatctctga agtgtacgag    1080

agcgagagag atgaagacgg cttcctgtac atggtctatg cctcccagga gacgttcggg    1140

ggatccatgg cctcctccga ggacgtcatc aaggagttca tgcgcttcaa ggtgcgcatg 1200

gagggctccg tgaacggcca cgagttcgag atcgagggcg agggcgaggg ccgcccctac 1260

gagggcaccc agaccgccaa gctgaaggtg accaagggcg gccccctgcc cttcgcctgg 1320

gacatcctgt cccctcagtt ccagtacggc tccaaggcct acgtgaagca ccccgccgac 1380

atccccgact acttgaagct gtccttcccc gagggcttca agtgggagcg cgtgatgaac 1440

ttcgaggacg gcggcgtggt gaccgtgacc caggactcct ccctgcagga cggcgagttc 1500

atctacaagg tgaagctgcg cggcaccaac ttcccctccg acggccccgt aatgcagaag 1560

aagaccatgg gctgggaggc ctccaccgag cggatgtacc ccgaggacgg cgccctgaag 1620

ggcgagatca agatgaggct gaagctgaag gacggcggcc actacgacgc cgaggtcaag 1680

accacctaca tggccaagaa gcccgtgcag ctgcccggcg cctacaagac cgacatcaag 1740

ctggacatca cctcccacaa cgaggactac accatcgtgg aacagtacga gcgcgccgag 1800

ggccgccact ccaccggcgc catcgatgaa ttgaattcca tgccgtccga gaagaccttc 1860

aaacagcgcc ggagcttcga acaaagagtg gaagatgtcc ggctcatccg ggagcagcac 1920

cccaccaaga tcccagtgat tatagagcga tacaagggtg agaagcagct gcccgtcctg 1980

gacaagacca agttccttgt acctgatcac gtgaatatga gcgaactcat caagataatt 2040

agaaggcgcc tgcagctcaa tgctaaccaa gccttcttcc tcctggtgaa tgggcacagc 2100

atggtgagtg tgtccacacc catctctgaa gtgtacgaga gcgagagaga tgaagacggc 2160

ttcctgtaca tggtctatgc ctcccaggag acgttctaa 2199

<210> 2
<211> 1818
<212> DNA
<213> Artificial Sequence

<220>
<223> Artificial Sequence

<400> 2
atggtgagca agggcgagga gctgttcacc ggggtggtgc ccatcctggt cgagctggac 60

ggcgacgtaa acggccacaa gttcagcgtg tccggcgagg gcgagggcga tgccacctac 120

ggcaagctga ccctgaagtt catctgcacc accggcaagc tgcccgtgcc ctggcccacc 180

ctcgtgacca ccctgaccta cggcgtgcag tgcttcagcc gctaccccga ccacatgaag 240

cagcacgact tcttcaagtc cgccatgccc gaaggctacg tccaggagcg caccatcttc 300

ttcaaggacg acggcaacta caagacccgc gccgaggtga agttcgaggg cgacaccctg 360

gtgaaccgca tcgagctgaa gggcatcgac ttcaaggagg acggcaacat cctggggcac 420

aagctggagt acaactacaa cagccacaac gtctatatca tggccgacaa gcagaagaac 480

ggcatcaagg tgaacttcaa gatccgccac aacatcgagg acggcagcgt gcagctcgcc 540

gaccactacc agcagaacac ccccatcggc gacggccccg tgctgctgcc cgacaaccac 600

tacctgagca cccagtccgc cctgagcaaa gaccccaacg agaagcgcga tcacatggtc 660

ctgctggagt tcgtgaccgc cgccgggatc actctcggca tggacgagct gtacaagggt 720

ggcggtggct cgggcggtgg tgggtcgggt ggcggcggat ctatcgatga attgaattcc 780

atgccgtccg agaagacctt caaacagcgc cggagcttcg aacaaagagt ggaagatgtc 840

cggctcatcc gggagcagca ccccaccaag atcccagtga ttatagagcg atacaagggt 900

gagaagcagc tgcccgtcct ggacaagacc aagttccttg tacctgatca cgtgaatatg 960

agcgaactca tcaagataat tagaaggcgc ctgcagctca atgctaacca agccttcttc 1020

ctcctggtga atgggcacag catggtgagt gtgtccacac ccatctctga agtgtacgag 1080

agcgagagag atgaagacgg cttcctgtac atggtctatg cctcccagga gacgttcggg 1140

atggcctcct ccgaggacgt catcaaggag ttcatgcgct tcaaggtgcg catggagggc 1200

tccgtgaacg gccacgagtt cgagatcgag ggcgagggcg agggccgccc ctacgagggc 1260

acccagaccg ccaagctgaa ggtgaccaag ggcggccccc tgcccttcgc ctgggacatc 1320

ctgtcccctc agttccagta cggctccaag gcctacgtga agcaccccgc cgacatcccc 1380

gactacttga agctgtcctt ccccgagggc ttcaagtggg agcgcgtgat gaacttcgag 1440

gacggcggcg tggtgaccgt gacccaggac tcctccctgc aggacggcga gttcatctac 1500

aaggtgaagc tgcgcggcac caacttcccc tccgacggcc ccgtaatgca gaagaagacc 1560

atgggctggg aggcctccac cgagcggatg taccccgagg acggcgccct gaagggcgag 1620

atcaagatga ggctgaagct gaaggacggc ggccactacg acgccgaggt caagaccacc 1680

tacatggcca agaagcccgt gcagctgccc ggcgcctaca agaccgacat caagctggac 1740

atcacctccc acaacgagga ctacaccatc gtggaacagt acgagcgcgc cgagggccgc 1800

cactccaccg gcgcctaa 1818

## Description

### Technical Field

The present invention relates to a fusion protein suitable for measuring autophagy, a nucleic acid having a base sequence coding for the fusion protein, a vector containing the nucleic acid, a cell or a transgenic non-human organism to which at least one of the nucleic acid and the vector has been introduced, and a method for measuring autophagy.

### Background Art

Autophagy is a representative intracellular degradation system where cytosolic components surrounded by autophagosomes are transported to lysosomes, where the cytosolic components are degraded. The autophagy has numerous functions such as processing of intracellular defects or wastes, nutritional self-sufficiency in a starvation state, embryonic development, prevention of neurodegeneration or cancer, and degradation of bacteria which have invaded cells. Recently, abnormalities in autophagy-related genes were found to contribute to neurodegenerative diseases. Therefore, the autophagy has also attracted attention as a target for drug development.

In basic and applied studies for autophagy, it is necessary and important to quantitatively evaluate an autophagy activity.

However, most of previous methods are complicated and non-quantitative such as counting the number of autophagosomes under a microscopy or detecting molecular modification associated with the autophagy.

There has been proposed a method in which an amount of a substrate degraded by the autophagy is measured to determine the autophagy activity (see, e.g., NPL 1). Specifically, a LC3 protein serving as the substrate is fused to a fluorescent protein to form a fusion protein and the total amount of the fusion protein is quantitated by flow cytometry. According to the method, the autophagy is capable of being determined to be activated when the total amount of the fusion protein is decreased.

However, the method is problematic in that fluctuation in an amount of LC3 protein synthesis is not considered. That is, it is not capable of being determined whether the amount of the LC3 protein is decreased by activation of the autophagy or by inhibition of the LC3 protein synthesis. This is a significant problem when measurement for a long period of time is required such as in screening of drugs for autophagy.

There also has been proposed a method in which two kinds of cells are used to measure the autophagy activity (see, e.g., NPL 2). Specifically, cells expressing a LC3 protein to be degraded by the autophagy in the form of a fusion protein with a tag and cells expressing a LC3 protein mutant not to be degraded by the autophagy in the form of a fusion protein with a tag are used. Amounts of both the tags are measured and thus a difference between the amounts is calculated to determine the autophagy activity. According to the method, it is considered that the autophagy activity is capable of being measured in view of fluctuation in the amount of LC3 protein synthesis.

However, the method is problematic in terms of complexity due to necessity of two kinds of cells and incorrect comparison due to intercellular variation in expression levels of the tags.

Therefore, a method for measuring the autophagy activity in a precise, quantitative, and simple manner has not been provided. As a result, keen demand has arisen for promptly providing the method.

### Citation List

### Non-Patent Literature

NPL 1: Shvets E, et al., Utilizing flow cytometry to monitor autophagy in living mammalian cells., Autophagy., 2008 Jul; 4 (5): 621 - 628
NPL 2: Farkas T, et al., Identification of novel autophagy regulators by a luciferase-based assay for the kinetics of autophagic flux, Autophagy., 2009 Oct; 5 (7): 1018 - 1025

### Summary of Invention

### Technical Problem

The present invention aims to solve the above existing problems and achieve the following object. That is, the present invention has an object to provide: a method for measuring autophagy by which an autophagy activity is capable of being measured in a precise, quantitative, and simple manner; a fusion protein suitable for measuring autophagy; a nucleic acid having a base sequence coding for the fusion protein; a vector containing the nucleic acid; and a cell or a transgenic non-human organism to which at least one of the nucleic acid and the vector has been introduced.

### Solution to Problem

Means for solving the above problems are as follows.
<1> A fusion protein including:
   a LC3 protein which includes a first tag; and
   a protein which includes a second tag,
   the LC3 protein being fused to the protein.
<2> A nucleic acid including
   a base sequence coding for the fusion protein according to <1>.
<3> A vector including
   the nucleic acid according to <2>.
<4> A cell including
   at least one of the nucleic acid according to <2> and the vector according to <3>,
   wherein the at least one of the nucleic acid and the vector being introduced into the cell.
<5> A transgenic non-human organism including
   at least one of the nucleic acid according to <2> and the vector according to <3>,
   wherein the at least one of the nucleic acid and the vector being introduced into the transgenic non-human organism.
<6> A method for measuring autophagy, the method including
   using at least one of the cell according to <4> and the transgenic non-human organism according to <5>.

### Advantageous Effects of Invention

According to the present invention, it is possible to solve the above existing problems and achieve the above object. The present invention can provide a method for measuring autophagy by which an autophagy activity is capable of being measured in a precise, quantitative, and simple manner; a fusion protein suitable for measuring autophagy; a nucleic acid having a base sequence coding for the fusion protein; a vector containing the nucleic acid; and a cell or a transgenic non-human organism to which at least one of the nucleic acid and the vector has been introduced.

### Brief Description of Drawings

FIG. 1 is an image illustrating results of western blotting in Example 2.
FIG. 2A is a graph illustrating measurement and analysis results of GFP fluorescence intensities in Example 3.
FIG. 2B is a graph illustrating measurement and analysis results of RFP fluorescence intensities in Example 3.
FIG. 2C is a graph illustrating ratios of GFP fluorescence intensities to RFP fluorescence intensities in Example 3.
FIG. 3 is a GFP / RFP ratio image in Example 4.
FIG. 4A is whole-body images of zebrafish 52 hours after fertilization in Example 5.
FIG. 4B is an enlarged image of a tail 24 hours after fertilization in Example 5 and illustrates a GFP fluorescence signal.
FIG. 4C is an enlarged image of a tail 24 hours after fertilization in Example 5 and illustrates a RFP fluorescence signal.
FIG. 4D is an enlarged image of a tail 24 hours after fertilization in Example 5 and illustrates a differential interference image.
FIG. 4E is an enlarged image of a tail 24 hours after fertilization in Example 5 and illustrates a GFP/RFP ratio image.
FIG. 5 is a GFP/RFP ratio image in Example 7.
FIG. 6A is an explanatory drawing illustrating a region in a zebrafish embryo at which images are taken in Example 8.
FIG. 6B is images illustrating a cell mass and a yolk of a zebrafish embryo taken in the same vision of field every 10 min since 2.4 hours after fertilization in Example 8.
FIG. 6C is a graph illustrating GFP/RFP ratios in Example 8.
FIG. 7A is a graph illustrating measurement results of GFP fluorescence intensities in fibroblasts expressing a fusion protein of a LC3 protein which includes a first tag and a LC3 protein mutant which includes a second tag and in which glycine at the carboxyl-terminus of the LC3 protein is deleted in Example 9.
FIG. 7B is a graph illustrating measurement results of RFP fluorescence intensities in fibroblasts expressing a fusion protein of a LC3 protein which includes a first tag and a LC3 protein mutant which includes a second tag and in which glycine at the carboxyl-terminus of the LC3 protein is deleted in Example 9.
FIG. 7C is a graph illustrating measurement results of GFP and RFP fluorescence intensities in fibroblasts expressing a fusion protein of a LC3 protein which includes a first tag and a LC3 protein mutant which includes a second tag and in which glycine at the carboxyl-terminus of the LC3 protein is deleted in the case of culturing under an enrichment condition in Example 9.
FIG. 7D is a graph illustrating measurement results of GFP and RFP fluorescence intensities in fibroblasts expressing a fusion protein of a LC3 protein which includes a first tag and a LC3 protein mutant which includes a second tag and in which glycine at the carboxyl-terminus of the LC3 protein is deleted in the case of culturing under a starvation condition in Example 9.
FIG. 7E is a graph illustrating measurement results of GFP fluorescence intensities in fibroblasts expressing a fusion protein of a LC3 protein which includes a first tag and a second tag in Example 9.
FIG. 7F is a graph illustrating measurement results of RFP fluorescence intensities in fibroblasts expressing a fusion protein of a LC3 protein which includes a first tag and a second tag in Example 9.
FIG. 7G is a graph illustrating measurement results of GFP and RFP fluorescence intensities in fibroblasts expressing a fusion protein of a LC3 protein which includes a first tag and a second tag in the case of culturing under an enrichment condition in Example 9.
FIG. 7H is a graph illustrating measurement results of GFP and RFP fluorescence intensities in fibroblasts expressing a fusion protein of a LC3 protein which includes a first tag and a second tag in the case of culturing under a starvation condition in Example 9.

### Description of Embodiments

### (Fusion protein)

A fusion protein of the present invention includes a LC3 (which may be referred to as MAP1LC3 (Microtubule-associated protein light chain 3)) protein which includes a first tag and a protein which includes a second tag; and, if necessary, further includes other amino acid sequences.

The fusion protein is cleaved by an intracellular endogenous enzyme ATG4 into the LC3 protein which includes a first tag and the protein which includes a second tag.

The ATG4 recognizes glycine at the carboxyl-terminus of the LC3 protein and cleaves a bond between the glycine and the amino acid immediately downstream thereof. Therefore, the fusion protein is capable of producing equal numbers of molecules of the LC3 protein which includes a first tag and the protein which includes a second tag.

### <LC3 protein which includes first tag>

The LC3 protein which includes a first tag (hereinafter may be referred to as "substrate to be degraded by autophagy") is incorporated into an autophagosome and selectively degraded by autophagy.

### -LC3 protein-

The LC3 protein is a protein essential for autophagosome formation and is known as a protein selectively degraded by the autophagy. In order for the LC3 protein to be incorporated into the autophagosome, a glycine residue located at the carboxyl-terminus of the LC3 protein needs to bind to phosphatidylethanolamine. When the glycine residue is deleted, the LC3 protein no longer binds to the autophagosome and thus, is not degraded by the autophagy.

The LC3 protein is a generic name of a protein family. For example, LC3A, LC3B, LC3B2, LC3C, GABARAP, GABARAPL1, and GABARAPL2 are known mammalian LC3 proteins. In yeast, Atg8 is known as the LC3 protein. The LC3 protein of the present invention encompasses these proteins.

Information about amino acid sequences or base sequences of the LC3 proteins is readily available from public databases such as GenBank (NCBI).

For example, the LC3 proteins are deposited in GenBank under the following accession numbers:
Rat LC3A · · · Amino acid sequence: NP_955794.1, Base sequence: NM_199500.2;
Human LC3A · · · Amino acid sequence: NP_115903.1, Base sequence: NM_032514;
Rat LC3B · · · Amino acid sequence: NP_074058.2, Base sequence: NC_005118;
Human LC3B · · · Amino acid sequence: NP_073729.1, Base sequence: NG_029030;
Human LC3B2 · · · Amino acid sequence: NP_001078950.1, Base sequence: NM_001085481.1;
Human LC3C · · · Amino acid sequence: NP_001004343.1, Base sequence: NM_001004S4S;
Human GABARAP · · · Amino acid sequence: NP_009209.1, Base sequence: NM_007278.1;
Human GABARAPL1 · · · Amino acid sequence: NP_113600.1, Base sequence: NM_031412.2;
Human GABARAPL2 · · · Amino acid sequence: NP_009216.1, Base sequence: NM_007285.6; and
Yeast Atg8 · · · Amino acid sequence: NP_009475.1, Base sequence: NM_001178S18.1.

The LC3 protein may have a mutation at any position in the amino acid sequence thereof, as long as the LC3 protein is incorporated into the autophagosome. The mutation is not particularly limited and may be appropriately selected depending on the intended purpose. Examples thereof include substitution, insertion, deletion, and addition.

### -First tag-

The first tag is not particularly limited and may be appropriately selected from tags known in the art, as long as it is different from the second tag.

The tag is not particularly limited and may be appropriately selected depending on the intended purpose. Examples thereof include fluorescent proteins, luciferases, polyhistidine sequences, and Flag sequences.

Specific examples of the fluorescent proteins include green fluorescent proteins, red fluorescent proteins, blue fluorescent proteins, yellow fluorescent proteins, and cyan fluorescent proteins.

Specific examples of the luciferases include firefly luciferase and Renilla luciferase.

The first tag may be linked to the LC3 protein directly or via a linker. A length and a sequence of the linker are not particularly limited and may be appropriately selected depending on the intended purpose.

### <Protein which includes second tag>

The protein which includes a second tag (hereinafter may be referred to as "internal standard substance not to be degraded by autophagy") is not incorporated into the autophagosome or degraded by the autophagy.

The protein which includes a second tag is not particularly limited and may be appropriately selected depending on the intended purpose, as long as it contains a second tag.

The protein which includes a second tag may be a protein consisting of a second tag or a LC3 protein mutant which includes a second tag and in which glycine at the carboxyl-terminus of a LC3 protein is deleted or substituted (hereinafter may be referred to as "LC3 protein mutant which includes a second tag").

### -Second tag-

The second tag is not particularly limited and may be appropriately selected from tags known in the art, as long as it is different from the first tag.

The tag may be the same as those described for the first tag.

Combinations of the first tag with the second tag are not particularly limited and may be appropriately selected depending on the intended purpose, as long as they are different from each other. Examples of the combinations include combinations of tags having different lengths of amino acid sequences, combinations of tags having different electrophoretic mobilities, combinations of tags being different fluorescent proteins, and combinations of tags being different luciferases. Specific examples of the combinations include combinations of green fluorescent proteins with red fluorescent proteins and combinations of firefly luciferases with Renilla luciferases.

### -LC3 protein mutant which includes second tag-

The LC3 protein mutant in the LC3 protein mutant which includes a second tag is not particularly limited and may be appropriately selected depending on the intended purpose, as long as an amino acid located at the carboxyl-terminus thereof is not glycine. However, the LC3 protein mutant is preferably those in which glycine located at the carboxyl-terminus of the LC3 protein is deleted, in terms of having the most similar properties to those of the LC3 protein.

The LC3 protein mutant is not degraded by the autophagy because it does not contain a glycine residue at the carboxyl-terminus.

The LC3 protein mutant may have a mutation at any position except the carboxyl-terminus of the LC3 protein, as long as it is not incorporated into the autophagosome. However, the LC3 protein mutant preferably has the same sequence as the LC3 protein at all positions except the carboxyl-terminus.

A method for preparing the LC3 protein mutant is not particularly limited and may be appropriately selected depending on the intended purpose. Examples thereof include a method in which a base sequence coding for the glycine located at the carboxyl-terminus of the LC3 protein is substituted with a stop codon or a base sequence coding for any amino acid except glycine.

The second tag may be linked to the LC3 protein mutant directly or via a linker. A length and a sequence of the linker are not particularly limited and may be appropriately selected depending on the intended purpose.

The LC3 protein mutant which includes a second tag preferably includes the second tag and the LC3 protein mutant in which glycine at the carboxyl-terminus of the LC3 protein is deleted or substituted in this order from the amino-terminus to the carboxyl-terminus.

### <Other amino acid sequences>

The other amino acid sequences are not particularly limited and may be appropriately selected depending on the intended purpose, as long as they do not impair effects of the present invention.

### <Aspect>

An aspect of the fusion protein is not particularly limited and may be appropriately selected depending on the intended purpose, as long as it does not impair effects of the present invention. However, an aspect in which the fusion protein includes the first tag, the LC3 protein, and the protein which includes a second tag in this order from the amino-terminus to the carboxyl-terminus.

A linker may be present between the first tag and the LC3 protein or between the LC3 protein and the protein which includes a second tag.

Specific examples of a base sequence coding for the fusion protein including the first tag, the LC3 protein, the second tag, and the LC3 protein mutant in which glycine at the carboxyl-terminus of the LC3 protein is deleted or substituted in this order from the amino-terminus to the carboxyl-terminus include that set forth in SEQ ID NO:1.

Specific examples of a base sequence coding for the fusion protein including the first tag, the LC3 protein, and the second tag in this order from the amino-terminus to the carboxyl-terminus include that set forth in SEQ ID NO:2.

A method for producing the fusion protein is not particularly limited and may be appropriately selected from methods known in the art. For example, the fusion protein may be expressed by inserting a nucleic acid having a base sequence coding for the fusion protein into a vector and introducing the vector into a cell.

According to the fusion protein of the present invention, the substrate to be degraded by autophagy is always capable of being synthesized in the same number of molecules as the internal standard substance not to be degraded by autophagy. Therefore, a ratio of the substrate to be degraded by autophagy to the internal standard substance not to be degraded by autophagy is not affected by synthesis and purely reflects an activity of autophagic degradation. That is, the autophagy activity is capable of being estimated by quantitatively calculating a remaining ratio of the first tag to the second tag.

Therefore, the fusion protein of the present invention is capable of being suitably used as a probe in a method for measuring autophagy by which an autophagy activity, which has been difficult to measure hitherto, is capable of being measured in a precise, quantitative, and simple manner.

### (Nucleic acid)

A nucleic acid of the present invention has a base sequence coding for the fusion protein of the present invention; and, if necessary, further has other base sequences.

The nucleic acid is not particularly limited and may be appropriately selected depending on the intended purpose, as long as it has the base sequence coding for the fusion protein of the present invention. The nucleic acid may be DNA or RNA.

The other base sequences are not particularly limited and may be appropriately selected depending on the intended purpose, as long as they do not impair effects of the present invention. Examples thereof include promoter sequences linked upstream of the nucleic acid coding for the fusion protein and terminator sequences linked downstream of the nucleic acid coding for the fusion protein.

The promoter sequences and the terminator sequences are not particularly limited and may be appropriately selected from sequences known in the art. Examples thereof include cytomegalovirus (CMV) promoter sequences.

A method for preparing the nucleic acid is not particularly limited and may be appropriately selected from methods known in the art. For example, the nucleic acid may be prepared through chemical synthesis or PCR.

The nucleic acid of the present invention is capable of being suitably used for producing the below-described cell or transgenic non-human organism of the present invention.

### (Vector)

A vector of the present invention includes the nucleic acid of the present invention; and, if necessary, further includes other base sequences.

The vector is not particularly limited and may be appropriately selected depending on the intended purpose, as long as it includes the nucleic acid of the present invention.

A vector to which the nucleic acid of the present invention is inserted is not particularly limited and may be appropriately selected from vectors known in the art. Examples thereof include plasmid vectors and virus vectors.

Specific examples of the vector include a pMRX-IP vector.

The other base sequences are not particularly limited and may be appropriately selected depending on the intended purpose, as long as they do not impair effects of the present invention. Examples thereof include promoter sequences linked upstream of the nucleic acid coding for the fusion protein and terminator sequences linked downstream of the nucleic acid coding for the fusion protein.

The promoter sequences and the terminator sequences are not particularly limited and may be appropriately selected from sequences known in the art. Examples thereof include cytomegalovirus (CMV) promoter sequences.

A method for inserting the nucleic acid of the present invention to the vector is not particularly limited and may be appropriately selected from methods known in the art. For example, the nucleic acid may be ligated to a restriction enzyme-cleaved site of the vector.

The vector of the present invention is capable of being suitably used for producing the below-described cell or transgenic non-human organism of the present invention.

### (Cell)

A cell of the present invention includes at least one of the nucleic acid of the present invention and the vector of the present invention which has been introduced thereto.

The cell is not particularly limited and may be appropriately selected depending on the intended purpose. Examples thereof include fibroblasts and HeLa cells.

A method for introducing the at least one of the nucleic acid of the present invention and the vector of the present invention is not particularly limited and may be appropriately selected from methods known in the art. For example, commercially available transfection reagents may be used.

A method for verifying that at least one of the nucleic acid and the vector has been successfully introduced into the cell is not particularly limited and may be appropriately selected from methods known in the art. Examples thereof include a method in which the tag in the cell is detected and a method in which DNA extracted from the cell is assayed by PCR.

The cell may be or may not be derived from a single clone, but is preferably derived from a single clone.

The cell of the present invention expresses the fusion protein of the present invention. As a result, the autophagy activity in the cell is capable of being objectively quantitated. Therefore, the cell of the present invention enables identification of cellular conditions affecting the autophagy activity, identification of genes involved in regulation of the autophagy, or identification of compounds affecting the autophagy activity.

### (Transgenic non-human organism)

A transgenic non-human organism of the present invention includes at least one of the nucleic acid of the present invention and the vector of the present invention which has been introduced thereto.

The transgenic non-human organism is not particularly limited and may be appropriately selected depending on the intended purpose. Examples thereof include animals, plants, and fungi.

Examples of the animals include mammals and fish. Specific examples of the mammals include mouse, rat, hamster, guinea pig, cat, dog, rabbit, goat, sheep, pig, and cattle. Specific examples of the fish include zebrafish, killifish, and goldfish.

Examples of the plants include Arabidopsis.

Examples of the fungi include yeast.

A method for producing the transgenic non-human organism is not particularly limited and may be appropriately selected from methods known in the art.

For example, the transgenic non-human animal may be produced by introducing at least one of the nucleic acid and the vector into a fertilized egg of a non-human animal and implanting the fertilized egg into a female of the non-human animal. A method for introducing is not particularly limited and may be appropriately selected depending on the intended purpose. Examples thereof include microinjection.

Examples of a method for introducing at least one of the nucleic acid and the vector into the plant include use of bacteria of the genus Agrobacterium, electroporation, particle gun, and microinjection. Examples of a method for introducing at least one of the nucleic acid and the vector into the fungi include spheroplast and electroporation.

A method for verifying that at least one of the nucleic acid and the vector has been successfully introduced into the transgenic non-human organism is not particularly limited and may be appropriately selected from methods known in the art. Examples thereof include a method in which the tag in the transgenic non-human organism is detected and a method in which DNA is extracted from the transgenic non-human organism and assayed by PCR.

The transgenic non-human organism preferably expresses the fusion protein of the present invention in a whole body.

The transgenic non-human organism of the present invention expresses the fusion protein of the present invention. This enables evaluation of the autophagy activity in vivo. Therefore, the transgenic non-human organism of the present invention can be used to identify individual conditions affecting the autophagy activity, to identify genes involved in regulation of the autophagy, or to identify compounds affecting the autophagy activity.

### (Method for measuring autophagy)

A method for measuring autophagy of the present invention uses at least one of the cell of the present invention and the transgenic non-human organism of the present invention.

In the method for measuring autophagy, the autophagy activity is estimated by quantitatively calculating a remaining ratio of the first tag to the second tag. According to the method for measuring autophagy, the autophagy activity, which has been difficult to measure hitherto, is capable of being measured in a simple, precise, and quantitative manner.

A method for quantitating the remaining ratio of the first tag to the second tag is not particularly limited and may be appropriately selected depending on the tags from methods known in the art. Examples thereof include western blotting, flow cytometry, fluorescence microscopy, a fluorescence plate reader method, and a dual luciferase assay.

For example, when tags having different lengths are used as the tags, western blotting is capable of being used; when different fluorescent proteins are used as the tags, flow cytometry, fluorescence microscopy, or a fluorescence plate reader method is capable of being used; or when different luciferases are used as the tags, a dual luciferase assay is capable of being used.

Among the methods for quantitating the remaining ratio of the first tag to the second tag, the fluorescence plate reader method is preferable because this method is capable of readily distinguishing the case where cells are killed due to administration of a certain drug from the case where fluorescent proteins are degraded, these cases having been impossible to distinguish with the previous methods.

The fluorescence microscopy also enables measurement, through imaging, of change in the autophagy activity over time, cell by cell, or tissue by tissue.

A target to be measured in the method for measuring autophagy is not particularly limited and may be appropriately selected depending on the intended purpose. Examples thereof include the cell or transgenic non-human organism of the present invention under a normal state and the cell or transgenic non-human organism of the present invention under an autophagy-induced state.

A method for inducing the autophagy is not particularly limited and may be appropriately selected depending on the intended purpose. Examples thereof include methods for starving the cell or the transgenic non-human organism.

The methods for starving are not particularly limited and may be appropriately selected depending on the intended purpose. For example, the cell or the transgenic non-human organism may be cultured in a starvation medium.

The cell or the transgenic non-human organism under each of the states may be administered with a test substance or environmental conditions of the cell or the transgenic non-human organism under each of the states may be changed.

The test substance is not particularly limited and may be appropriately selected depending on the intended purpose. Examples thereof include drugs.

The environmental conditions are not particularly limited and may be appropriately selected depending on the intended purpose. Examples thereof include a seeding density of the cell and a temperature.

For example, substances or environmental conditions affecting the autophagy activity or genes involved in regulation of the autophagy are capable of bering identified by (1) comparing an autophagy activity of the cell or transgenic non-human organism of the present invention under the normal state with an autophagy activity of the cell or transgenic non-human organism of the present invention under the autophagy-induced state; (2) comparing an autophagy activity of the cell or transgenic non-human organism of the present invention under the normal state with an autophagy activity of the cell or transgenic non-human organism of the present invention under the same state as the normal state except administration of the test substance or change in the environmental conditions; or (3) comparing an autophagy activity of the cell or transgenic non-human organism of the present invention under the autophagy-induced state with an autophagy activity of the cell or transgenic non-human organism of the present invention under the same state as the autophagy-induced state except administration of the test substance or change in the environmental conditions.

### Examples

The present invention will now be described in detail with reference to Examples described below, but the present invention is not limited thereto in any way.

### (Example 1: Establishment of fibroblast cell line; with cloning)

A fibroblast cell line expressing a fusion protein of a LC3 protein which included a first tag and a LC3 protein which included a second tag and in which glycine at the carboxyl-terminus of the LC3 protein was deleted was established in the following manner.

Nucleic acids coding for the first tag, the LC3 protein, the second tag, and the LC3 protein in which glycine at the carboxyl-terminus of a LC protein was deleted were as described below.

### -First tag-

Green fluorescent protein (hereinafter may be referred to as "EGFP" or "GFP") gene.

### -LC3 protein-

Rat LC3B cDNA (from 1st to 360th bases).

### -Second tag-

Monomeric red fluorescent protein (hereinafter may be referred to as "mRFP" or "RFP") gene.

### -LC3 protein in which glycine at carboxyl-terminus of LC3 protein is deleted-

Rat LC3B cDNA (from 1st to 360th bases) in which 358th to 360th bases "ggg" were substituted with "taa (stop codon)."

The nucleic acids coding for the first tag, the LC3 protein, the second tag, and the LC3 protein in which glycine at the carboxyl-terminus of the LC protein was deleted were inserted in this order into a BamHI site of a retrovirus vector pMRX-IP (Saitoh, H., Nakano, H., Yamamoto, N. and Yamaoka, S.: NEMO-independent NF-kB Activation through the Lymphotoxin-beta Receptor Signaling. FEBS Letters, 532, 45 - 51, 2002).

Note that, a base sequence of a nucleic acid containing nucleic acids coding for the first tag, the LC3 protein, the second tag, and the LC3 protein in which glycine at the carboxyl-terminus of a LC protein was deleted in this order is set forth in SEQ ID NO:1. In SEQ ID NO:1, a base sequence of from 1st to 717th bases codes for the first tag, a base sequence of from 781st to 1,140th bases codes for the LC3 protein, a base sequence of from 1,147th to 1,821st bases codes for the second tag, and a base sequence of from 1,840th to 2,199th bases codes for the LC3 protein in which glycine at the carboxyl-terminus of a LC protein was deleted. Note that, in SEQ ID NO:1, base sequences of from 718th to 780th, from 1,141st to 1,146th, and from 1,822nd to 1,839th bases code for amino acids serving as linkers.

Plat-E cells were transfected with the pMRX-IP vectors using FUGENE (registered trademark) HD (available from Roche). Three days after transfection, viral particles released into a culture supernatant were collected. The supernatant containing the viral particles and 8 µg/mL of polybrene were added to fibroblasts (established from wild-type mice) and incubated overnight. Thus, the gene was introduced into the fibroblasts.

Gene-expressing cells were selected with puromycin and then a single clone was isolated. Thus, a fibroblast cell line expressing the fusion protein of a LC3 protein which included a first tag and a LC3 protein mutant which included a second tag and in which glycine at the carboxyl-terminus of the LC3 protein was deleted was obtained.

### (Example 2: Western blotting)

The fibroblasts obtained in Example 1 were cultured in a starvation medium (amino acid-free DMEM medium (available from Invitrogen)) for 0 hours, 1 hour, 2 hours, 6 hours, or 12 hours to thereby induce autophagy.

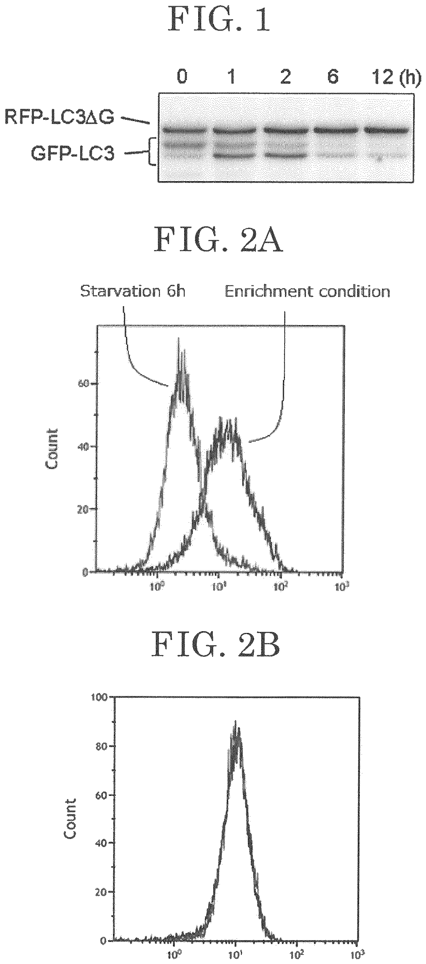

The cells which had been cultured in the starvation medium for the predetermined times were lysed with a lysis buffer (50 mM Tris-HCl (pH 7.5), 150 mM NaCl, 1 mM EDTA, 1% Triton X-100, 10 mM NaF, 0.4 mM Na₃VO₄, 10 mM sodium pyrophosphate, a protease inhibitor (Complete) (available from Roche)), and centrifuged at 15,000 rpm for 15 min to collect supernatant. The supernatant was added with a 6 x sample buffer (280 mM Tris-HCl (pH 6.8), 30% Glycerol, 10% SDS, 9.3% DTT, 0.1% BPB) in one-sixth volume thereof and then boiled for 5 min. Then, the resultant sample was separated by SDS-PAGE and transferred onto a polyvinylidene fluoride (PVDF) membrane. Then, western blotting was performed with an anti-LC3 antibody (Quy et al. J Biol Chem. 288: 1125 - 34, 2013.) and an HRP-conjugated anti-rabbit IgG antibody (available from Jackson ImmunoResearch). Thereafter, IMMOBILON WESTERN (available from Millipore) was used to emit fluorescence and LAS-3000MINI (available from Fujifilm Corporation) was used to detect the fluorescent signal. Results are presented in FIG. 1.

In FIG. 1, numerals 0, 1, 2, 6, and 12 denote culture times in the starvation medium, RFP-LC3ΔG denotes signals from the LC3 protein mutant which includes a second tag and in which glycine at the carboxyl-terminus of the LC3 protein is deleted, and GFP-LC3 denotes signals from the LC3 protein which includes a first tag.

It was confirmed from the results of FIG. 1 that the fusion protein of a LC3 protein which included a first tag and a LC3 protein mutant which included a second tag and in which glycine at the carboxyl-terminus of the LC3 protein was deleted was cleaved intracellularly into the LC3 protein which included a first tag and the LC3 protein mutant which included a second tag and in which glycine at the carboxyl-terminus of the LC3 protein was deleted.

Additionally, it was confirmed the RFP-LC3ΔG was stably present, whereas the GFP-LC3 was gradually degraded to be decreased in its amount as the autophagy was induced.

### (Example 3: Flow cytometry)

The fibroblasts obtained in Example 1 were washed with PBS and then cultured in a normal medium (10% FBS-containing DMEM (available from SIGMA), enrichment condition) or a starvation medium (amino acid-free DMEM (available from Invitrogen), starvation condition) for 6 hours.

After cultivation, the cells were harvested with 0.05% trypsin/EDTA, collected by centrifugation, suspended in 4% paraformaldehyde, and incubated on ice for 10 min. Thereafter, the thus-fixed cells were washed with cold PBS, centrifuged to remove the supernatant, and suspended in cold PBS again.

Fluorescence intensity of each cell was measured by a cell analyzer (EC800, available from Sony) and analyzed by an analysis software (Kaluza, Beckman Coulter). GFP and RFP were analyzed with lasers at 488 nm and 561 nm. Results are presented in FIGs. 2A to 2C.

FIG. 2A illustrates measurement and analysis results of fluorescence intensity of GFP serving as the first tag; and FIG. 2B illustrates measurement and analysis results of fluorescence intensity of RFP serving as the second tag. In FIGs. 2A and 2B, the horizontal axis represents the fluorescence intensity and the vertical axis represents the event count.

It was confirmed from the results of FIG. 2A that the fluorescence intensity of GFP was decreased under the starvation condition, indicating that the autophagy was induced. Whereas, it was confirmed from the results of FIG. 2B that the fluorescence intensity of RFP was not changed even under the starvation condition.

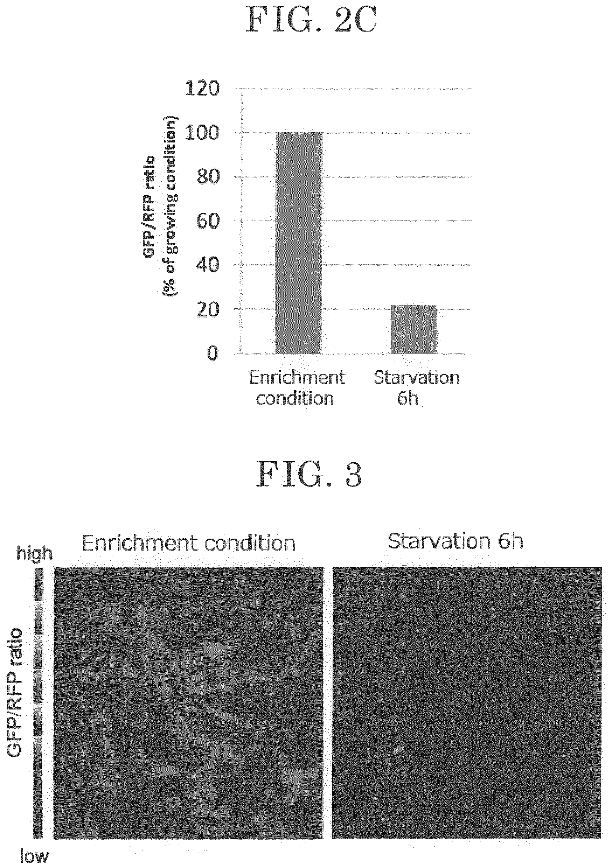

FIG. 2C illustrates a ratio of the fluorescence intensity of GFP to the fluorescence intensity of RFP (GFP/RFP) normalized so that the enrichment condition was 100%.

As illustrated in FIG. 2C, the GFP/RFP ratio was decreased by about 80% under the starvation condition compared to the enrichment condition. It was confirmed from the results of FIG. 2C that the cells expressing the fusion protein of the present invention enabled quantitative measurement of the autophagy activity.

### (Example 4: Fluorescence microscopy)

The fibroblasts obtained in Example 1 cultured in a 6-well plate were cultured in a normal medium (10% FBS-containing DMEM (available from SIGMA), enrichment condition) or a starvation medium (EBSS medium (available from Hyclone), starvation condition) for 6 hours and then fixed with 4% paraformaldehyde at room temperature for 10 min.

A fluorescence microscope (OLYMPUS IX81, available from Olympus Corporation) equipped with an objective lens (10×, available from Olympus Corporation) and a cooled CCD camera (COOLSNAPHQ₂, available from NIPPON ROPER K.K.) was used to detect a GFP fluorescence signal and a RFP fluorescence signal in the same field of vision under the following conditions: exposure time: 500 ms and binning: 2×.

An image processing software METAMORPH ver. 7 (available from Molecular Devices Japan K.K.) was used to generate a GFP/RFP ratio image. According to fluorescence microscopy, a GFP/RFP ratio is capable of displayed with a pseudo color and the autophagy activity is capable of being measured from the color. Results are presented in FIG. 3.

In FIG. 3, the left image illustrates a GFP/RFP ratio image under the enrichment condition and the right image illustrates a GFP/RFP ratio image under the starvation condition. Note that, a lower GFP/RFP ratio indicates a higher autophagy activity.

It was confirmed from the results of FIG. 3 that the LC3 protein mutant to which RFP was added as the second tag and in which glycine at the carboxyl-terminus of the LC3 protein was deleted was stably present, whereas the LC3 protein to which GFP was added as the first tag was gradually degraded to be decreased in its amount as the autophagy was induced.

### (Example 5: Production of transgenic zebrafish)

Zebrafish expressing the fusion protein of a LC3 protein which included a first tag and a LC3 protein which included a second tag and in which glycine at the carboxyl-terminus of the LC3 protein was deleted were produced in the following manner.

The nucleic acids coding for the first tag, the LC3 protein, the second tag, and the LC3 protein in which glycine at the carboxyl-terminus of the LC protein was deleted were the same as those used in Example 1.

The nucleic acids coding for the first tag, the LC3 protein, the second tag, and the LC3 protein in which glycine at the carboxyl-terminus of the LC protein was deleted were inserted in this order into an XhoI site of a pCS2 vector (Turner et al. Genes dev. 8: 1434 - 1447, 1994). The pCS2 vector was linearized with NotI, and then purified by phenol/chloroform treatment and ethanol precipitation. A mRNA of the nucleic acid coding for the first tag, the LC3 protein, the second tag, and the LC3 protein in which glycine at the carboxyl-terminus of the LC protein was deleted was synthesized by in vitro transcription using the thus-purified product serving as a template, a MMESSAGE MMACHINE (registered trademark) SP6 kit (available from Ambion), and a POLY(A) TAILING kit (available from Ambion). The mRNA was purified using a RNEASY MINI kit (available from Qiagen).

Zebrafish (strain: RIKEN RW, kept at 28°C under 14 h light/10 h dark cycles) were crossed to prepare fertilized eggs. About 100 pg of the mRNA was microinjected into each embryo at the one cell-stage under a stereoscopic microscope using FEMTOJET (available from Eppendorf) and FEMTOCHIP II (available from Eppendorf). Then, the embryos were cultured in an incubator at 28°C.

Twenty-four hours after fertilization, the embryos from which egg shells had been removed were transferred to a glass bottom dish (available from IWAKI). A confocal microscope (OLYMPUS FV1000-IX81, available from Olympus Corporation) equipped with an objective lens (10× or 30x, available from Olympus Corporation) and lasers (473 nm and 559 nm) was used to take a GFP fluorescence signal, a RFP fluorescence signal, and a differential interference image in the same field of vision under anesthesia (0.02% tricaine, available from Sigma). The image processing software METAMORPH ver. 7 (available from Molecular Devices Japan K.K.) was used to generate a GFP/RFP ratio image.

Additionally, a GFP fluorescence signal, a RFP fluorescence signal, and a differential interference image for the whole body of zebrafish 52 hours after fertilization were taken in the same field of vision.

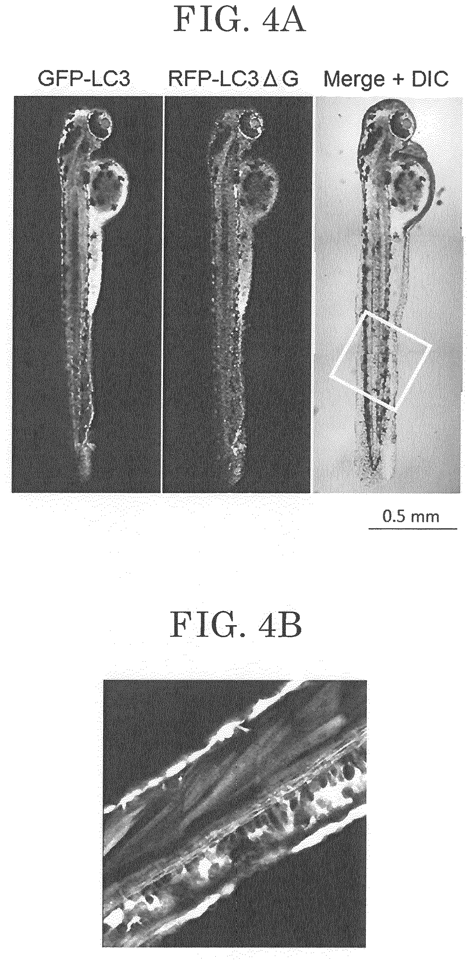

Results are presented in FIGs. 4A to 4E.

FIG. 4A illustrates whole body images of zebrafish 52 hours after fertilization where GFP-LC3 denotes the GFP fluorescence signal, RFP-LC3ΔG denotes the RFP fluorescence signal, and Merge + DIC denotes the differential interference image.

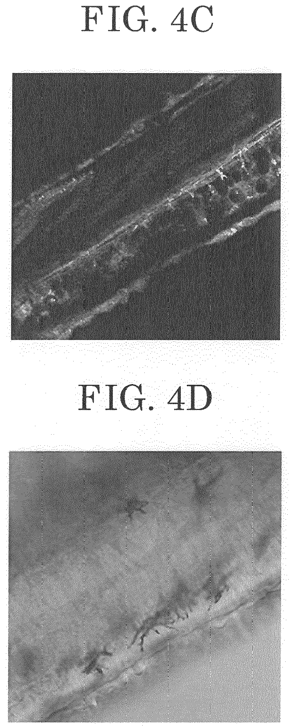

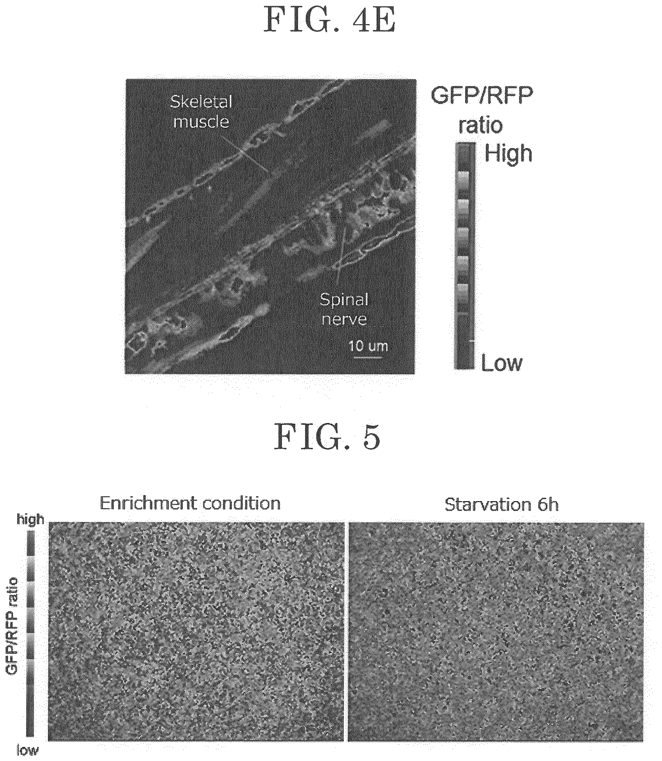

FIGs. 4B to 4E illustrate enlarged images of a tail 24 hours after fertilization. FIG. 4B illustrates the GFP fluorescence signal, FIG. 4C illustrates the RFP fluorescence signal, FIG. 4D illustrates the differential interference image, and FIG. 4E illustrates the GFP/RFP ratio image. Note that, a lower GFP/RFP ratio indicates a higher autophagy activity.

It was confirmed from the results of FIGs. 4A to 4E that the autophagy activity was capable of being measured in the zebrafish by expressing the fusion protein of a LC3 protein which included a first tag and a LC3 protein which included a second tag and in which glycine at the carboxyl-terminus of the LC3 protein was deleted. It was suggested in Example 5 that the autophagy activity was higher in the skeletal muscle than the spinal nerves.

### (Example 6: Establishment of HeLa cell line)

A HeLa cell line expressing a fusion protein of a LC3 protein which included a first tag and a LC3 protein which included a second tag and in which glycine at the carboxyl-terminus of the LC3 protein was deleted was established in the following manner.

The nucleic acids coding for the first tag, the LC3 protein, the second tag, and the LC3 protein in which glycine at the carboxyl-terminus of a LC protein was deleted were the same as described in Example 1. The pMRX-IP vector to which the nucleic acids coding for the first tag, the LC3 protein, the second tag, and the LC3 protein in which glycine at the carboxyl-terminus of a LC protein was deleted had been inserted in this order was prepared in the same manner as in Example 1.

HEK293T cells were transfected with the pMRX-IP vectors along with pCG-VSV-G and PCG-gag-pol using FUGENE (registered trademark) HD (available from Roche). Three days after transfection, viral particles released into a culture supernatant were collected. The supernatant containing the viral particles and 8 µg/mL of polybrene were added to HeLa cells and incubated overnight. Thus, the gene was introduced into the HeLa cells.

Gene-expressing cells were selected with puromycin. Thus, a HeLa cell line expressing the fusion protein of a LC3 protein which included a first tag and a LC3 protein mutant which included a second tag and in which glycine at the carboxyl-terminus of the LC3 protein was deleted was obtained.

### (Example 7: Fluorescence microscopy)

The HeLa cells obtained in Example 6 cultured in a 96-well plate were cultured in a normal medium (10% FBS-containing DMEM (available from SIGMA), enrichment condition) or a starvation medium (amino acid-free DMEM medium (available from Invitrogen), starvation condition) for 6 hours and then fixed with 4% paraformaldehyde at room temperature for 10 min.

A fluorescence microscope (OLYMPUS IX81, available from Olympus Corporation) equipped with an objective lens (4x, available from Olympus Corporation) and a cooled CCD camera (COOLSNAPHQ₂, available from NIPPON ROPER K.K.) was used to detect a GFP fluorescence signal and a RFP fluorescence signal in the same field of vision under the following conditions: exposure time: 1,000 ms and binning: 1×.

An image processing software METAMORPH ver. 7 (available from Molecular Devices Japan K.K.) was used to generate a GFP/RFP ratio image. According to fluorescence microscopy, a GFP/RFP ratio is capable of being displayed with a pseudo color and the autophagy activity is capable of being measured from the color. Results are presented in FIG. 5.

In FIG. 5, the left image illustrates a GFP/RFP ratio image under the enrichment condition and the right image illustrates a GFP/RFP ratio image under the starvation condition. Note that, a lower GFP/RFP ratio indicates a higher autophagy activity.

It was confirmed from the results of FIG. 5 that the LC3 protein mutant to which RFP was added as the second tag and in which glycine at the carboxyl-terminus of the LC3 protein was deleted was stably present, whereas the LC3 protein to which GFP was added as the first tag was gradually degraded to be decreased in its amount as the autophagy was induced.

### (Example 8: Production of fertilized egg of transgenic zebrafish)

Zebrafish expressing the fusion protein of a LC3 protein which included a first tag and a LC3 protein which included a second tag and in which glycine at the carboxyl-terminus of the LC3 protein was deleted were produced in the following manner.

The nucleic acids coding for the first tag, the LC3 protein, the second tag, and the LC3 protein in which glycine at the carboxyl-terminus of the LC protein was deleted were the same as those used in Example 1.

The nucleic acids coding for the first tag, the LC3 protein, the second tag, and the LC3 protein in which glycine at the carboxyl-terminus of the LC protein was deleted were inserted in this order into a BamHI site of a pT2AL200R150G vector (Genetics. 2006 Oct; 174 (2): 639 - 49. http://www.ncbi.nlm.nih.gov/pmc/articles/PMC1602067/) (hereinafter may be referred to as "pT2AL200R150G-GFP-LC3-RFP-LC3ΔG vector").

A transposase mRNA was synthesized by in vitro transcription using a pCS2-Transposase vector (Dev Cell. 2004 Jul; 7 (1): 133 - 44. http://www.ncbi.nlm.nih.gov/pubmed/15239961) serving as a template and a MMESSAGE MMACHINE (registered trademark) SP6 kit (available from Ambion). The mRNA was purified using a RNEASY MINI kit (available from Qiagen).

Zebrafish (strain: RIKEN RW, kept at 28°C under 14 h light/10 h dark cycles) were crossed to prepare fertilized eggs. About 100 pg of the pT2AL200R150G-GFP-LC3-RFP-LC3ΔG vector and about 100 pg of the transposase mRNA were microinjected into each of embryos at the one cell-stage under a stereoscopic microscope using FEMTOJET (available from Eppendorf) and FEMTOCHIP II (available from Eppendorf). Then, the embryos were grown in an incubator at 28°C.

Thereafter, the grown zebrafish were raised on brine shrimp and artificial feed (HIKARI LAB 130 and HIKARI LAB 450, available from MEITO system) for about 3 months.

Adult zebrafish, which were potentially transgenic, were crossed with wild-type zebrafish (strain: RIKEN RW). Twenty-four hours after fertilization, the resultant embryos were transferred to a glass bottom dish. A confocal microscope (OLYMPUS FV1000-IX81, available from Olympus Corporation) equipped with an objective lens (10× or 30×, available from Olympus Corporation) and lasers (473 nm and 559 nm) was used to observe the embryos under anesthesia (0.02% tricaine, available from Sigma). When both of a GFP signal and an RFP signal were observed, a parent of the embryo was determined as a transgenic zebrafish expressing the fusion protein of a LC3 protein which included a first tag and a LC3 protein which included a second tag and in which glycine at the carboxyl-terminus of the LC3 protein was deleted (hereinafter may be referred to as "GFP-LC3-RFP-LC3ΔG transgenic zebrafish").

### <Observation of fertilized egg>

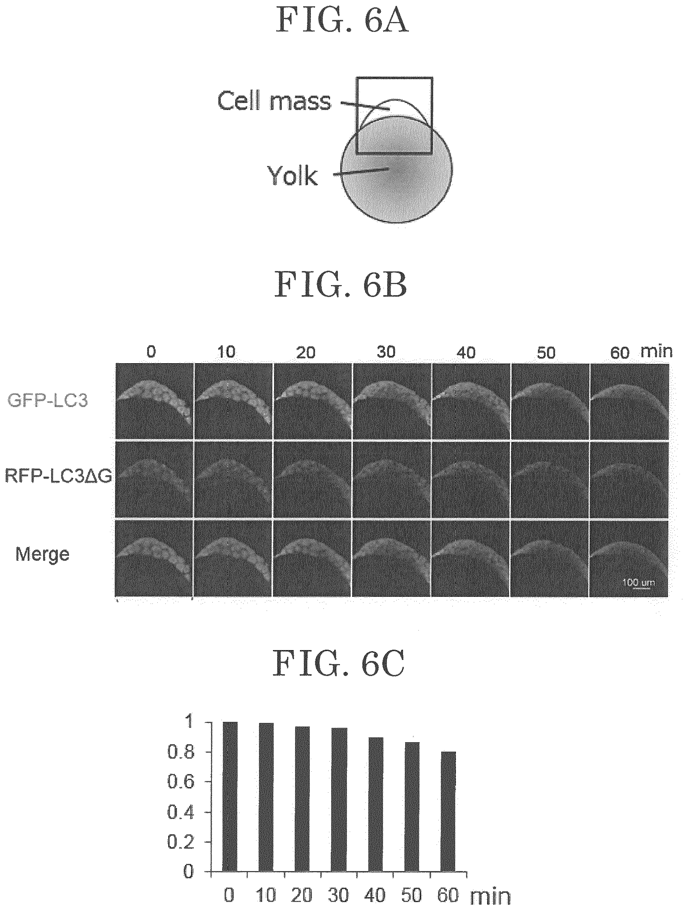

Female GFP-LC3-RFP-LC3ΔG transgenic zebrafish were crossed with male wild-type zebrafish (strain: RIKEN RW) to prepare fertilized eggs. Then, 2.4 hours after fertilization, the resultant embryos with egg shells were transferred to a glass bottom dish. A GFP fluorescence signal and an RFP fluorescence signal were taken in the same vision of field every 10 min. An image processing software IMAGEJ (free software) was used to quantify signals in a cell mass region. Based on the results, a GFP/RFP ratio was determined. Rates at each time point were plotted, assuming that the initial ratio is taken as 1. Results are presented in FIGs. 6A to 6C.

FIG. 6A is an explanatory drawing illustrating a region at which images in FIG. 6B are taken, where the region is surrounded by a rectangle in FIG. 6A.

FIG. 6B illustrates images of a cell mass and a yolk of a zebrafish embryo taken in the same vision of field every 10 min since 2.4 hours after fertilization. In this figure, GFP-LC3 denotes a GFP fluorescence signal, RFP-LC3ΔG denotes an RFP fluorescence signal, and Merge denotes merged GFP and RFP fluorescence signals.

FIG. 6C illustrates GFP/RFP ratios of GFP signals to RFP signals in the cell mass region. Note that, a lower GFP/RFP ratio indicates a higher autophagy activity.

It was confirmed from the results of FIGs. 6B and 6C that female GFP-LC3-RFP-LC3ΔG transgenic zebrafish were capable of being used to measure the autophagy activity physiologically induced after fertilization.

### (Example 9: Flow cytometry; without cloning)

A (1) fusion protein of a LC3 protein which included a first tag and a LC3 protein which included a second tag and in which glycine at the carboxyl-terminus of the LC3 protein was deleted was compared with a (2) fusion protein of a LC3 protein which included a first tag and a second tag using flow cytometry in the following manner.

### <Preparation of fibroblasts expressing (1) fusion protein of LC3 protein which includes first tag and LC3 protein mutant which includes second tag and in which glycine at the carboxyl-terminus of LC3 protein is deleted>

Fibroblasts (without cloning) expressing the fusion protein of a LC3 protein which included a first tag and a LC3 protein mutant which included a second tag and in which glycine at the carboxyl-terminus of the LC3 protein was deleted were prepared in the same manner as in Example 1, except that a single clone was not isolated.

### <Preparation of fibroblasts expressing (2) fusion protein of LC3 protein which includes first tag and second tag>

Fibroblasts (without cloning) expressing the fusion protein of a LC3 protein which included a first tag and a second tag were prepared in the same manner as in Example 1, except that the nucleic acids were changed to the following nucleic acids and a single clone was not isolated.

### [Nucleic acid]

### -First tag-

Green fluorescent protein (hereinafter may be referred to as "EGFP" or "GFP") gene.

### -LC3 protein-

Rat LC3B cDNA (from 1st to 360th bases).

### -Second tag-

Monomeric red fluorescent protein (hereinafter may be referred to as "mRFP" or "RFP") gene.

The nucleic acids coding for the first tag, the LC3 protein, and the second tag were inserted in this order into a BamHI site of a retrovirus vector pMRX-IP.

Note that, a base sequence of a nucleic acid containing nucleic acids coding for the first tag, the LC3 protein, and the second tag in this order is set forth in SEQ ID NO:2. In SEQ ID NO:2, a base sequence of from 1st to 717th bases codes for the first tag, and a base sequence of from 781st to 1,140th bases codes for the LC3 protein, and a base sequence of from 1,141st to 1,818th codes for the second tag. Note that, in SEPIA ID NO:2, a base sequence of from 718th to 780th bases codes for amino acids serving as a linker.

### <Flow cytometry>

The fibroblasts expressing the (1) fusion protein of a LC3 protein which included a first tag and a LC3 protein mutant which included a second tag and in which glycine at the carboxyl-terminus of the LC3 protein was deleted and the fibroblasts expressing the (2) fusion protein of a LC3 protein which included a first tag and a second tag were washed with PBS and then cultured in a normal medium (10% FBS-containing DMEM (available from SIGMA), enrichment condition) or a starvation medium (amino acid-free DMEM (available from Invitrogen), starvation condition) for 12 hours.

After cultivation, the cells were harvested with 0.05% trypsin/EDTA, collected by centrifugation, suspended in 4% paraformaldehyde, and incubated on ice for 10 min. Thereafter, the thus-fixed cells were washed with cold PBS, centrifuged to remove the supernatant, and suspended in cold PBS again.

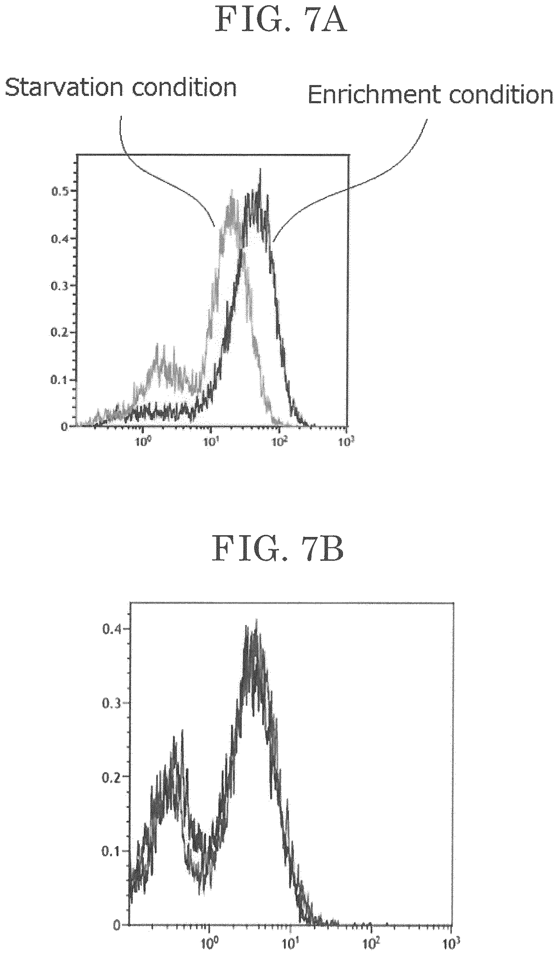

Fluorescence intensity of each cell was measured by a cell analyzer (EC800, available from Sony) and analyzed by an analysis software (Kaluza, Beckman Coulter). GFP and RFP were analyzed with lasers at 488 nm and 561 nm. Results are presented in FIGs. 7A to 7H.

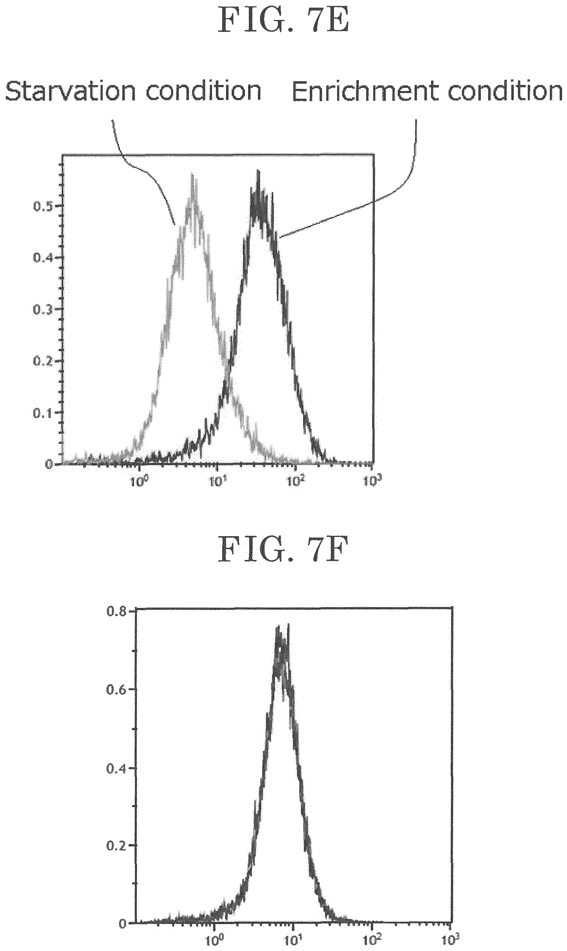

FIGs. 7A to 7D illustrate measurement results of the fibroblasts expressing the (1) fusion protein of a LC3 protein which includes a first tag and a LC3 protein mutant which includes a second tag and in which glycine at the carboxyl-terminus of the LC3 protein is deleted. FIGs. 7E to 7H illustrate measurement results of the fibroblasts expressing the (2) fusion protein of a LC3 protein which includes a first tag and a second tag.

FIGs. 7A and 7E illustrate measurement and analysis results of GFP fluorescence intensities; and FIGs. 7B and 7F illustrate measurement and analysis results of RFP fluorescence intensities. In FIGs. 7A, 7B, 7E, and 7F, the horizontal axis represents the fluorescence intensity and the vertical axis represents the event count.

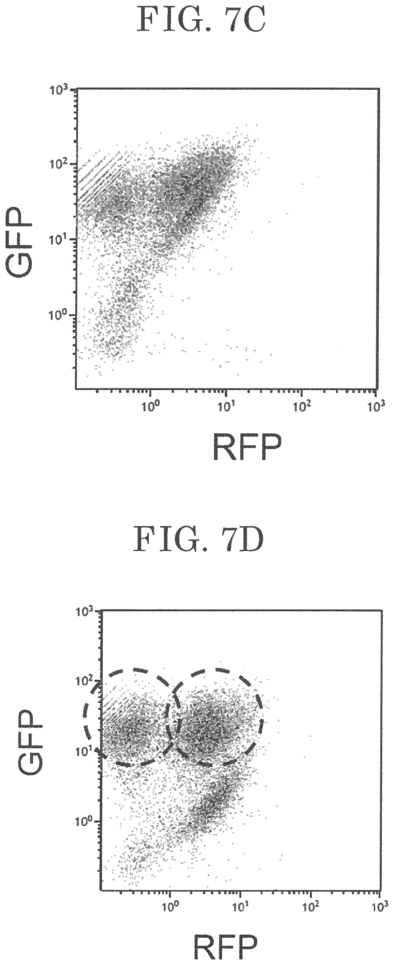

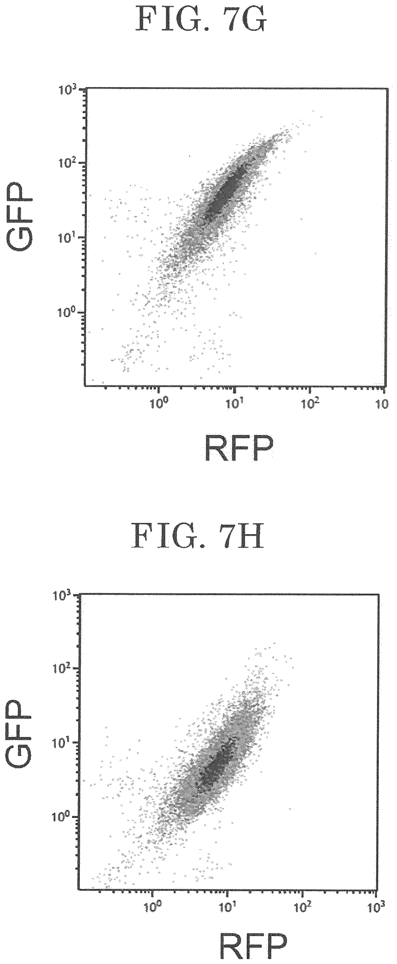

FIGs. 7C and 7G illustrate results in the case of culturing under the enrichment condition. FIGs. 7D and 7H illustrate results in the case of culturing under the starvation condition. In FIGs. 7C, 7D, 7G, and 7H, the horizontal axis represents the RFP fluorescence intensity and the vertical axis represents the GFP fluorescence intensity.

It was confirmed from the results of FIGs. 7A, 7B, 7E, and 7F that the GFP fluorescence intensity was decreased under the starvation condition, whereas the RFP fluorescence intensity did not change even under the starvation condition.

It was observed from the results of FIGs. 7G and 7H that the GFP fluorescence was selectively decreased in all of the fibroblasts expressing the (2) fusion protein of a LC3 protein which included a first tag and a second tag. Whereas, from the results of FIGs. 7C and 7D, populations containing "GFP-LC3 protein mutants" generated through homologous recombination between two LC3 protein sequences (regions surrounded by dotted lines in FIG. 7D) were observed in the fibroblasts expressing the (1) fusion protein of a LC3 protein which included a first tag and a LC3 protein mutant which included a second tag and in which glycine at the carboxyl-terminus of the LC3 protein was deleted. Therefore, it was found that cells in which the homologous recombination had not occurred were more preferably cloned.

Therefore, it was demonstrated that the (2) fusion protein of a LC3 protein which included a first tag and the second tag was more convenient than the (1) fusion protein of a LC3 protein which included a first tag and a LC3 protein mutant which included a second tag and in which glycine at the carboxyl-terminus of the LC3 protein was deleted.

From the above results, it was demonstrated that the remaining ratio of the first tag to the second tag enabled measurement of the autophagy activity in a precise, quantitative, and simple manner without being affected by fluctuation in an amount of LC3 protein synthesis.

The fusion protein, the nucleic acid, the vector, the cell, and the transgenic non-human organism of the present invention are capable of being suitably used for kits for measuring the autophagy activity or as means for screening methods in autophagy-related drug development.

Aspects of the present invention are as follows:
<1> A fusion protein including:
   a LC3 protein which includes a first tag; and
   a protein which includes a second tag,
   the LC3 protein being fused to the protein.
<2> The fusion protein according to <1>, wherein the fusion protein is cleaved by an intracellular endogenous enzyme ATG4 into the LC3 protein which includes a first tag and the protein which includes a second tag.
<3> The fusion protein according to <1> or <2>, wherein the fusion protein includes the first tag, the LC3 protein, and the protein which includes a second tag in this order from an amino-terminus to a carboxyl-terminus.
<4> The fusion protein according to any one of <1> to <3>, wherein the protein which includes a second tag consists of the second tag.
<5> The fusion protein according to any one of <1> to <3>, wherein the protein which includes a second tag is a LC3 protein mutant which includes the second tag and in which glycine at a carboxyl-terminus of the LC3 protein is deleted or substituted.
<6> The fusion protein according to <5>, wherein the fusion protein includes the second tag and the LC3 protein mutant in which glycine at a carboxyl-terminus of the LC3 protein is deleted or substituted in this order from the amino-terminus to the carboxyl-terminus.
<7> A nucleic acid including
   a base sequence coding for the fusion protein according to any one of <1> to <6>.
<8> A vector including
   the nucleic acid according to <7>.
<9> A cell including
   at least one of the nucleic acid according to <7> and the vector according to <8>,
   the at least one of the nucleic acid and the vector being introduced into the cell.
<10> A transgenic non-human organism including
   at least one of the nucleic acid according to <7> and the vector according to <8>,
   the at least one of the nucleic acid and the vector being introduced into the transgenic non-human organism.
<11> A method for measuring autophagy, the method including
   using at least one of the cell according to <9> and the transgenic non-human organism according to <10>.

## Claims

1. A fusion protein comprising:
a LC3 protein which includes a first tag; and
a protein which includes a second tag,
the LC3 protein being fused to the protein.

2. The fusion protein according to claim 1, wherein the fusion protein is cleaved by an intracellular endogenous enzyme ATG4 into the LC3 protein which includes a first tag and the protein which includes a second tag.

3. The fusion protein according to claim 1 or 2, wherein the fusion protein includes the first tag, the LC3 protein, and the protein which includes a second tag in this order from an amino-terminus to a carboxyl-terminus.

4. The fusion protein according to any one of claim 1 to 3, wherein the protein which includes a second tag consists of the second tag.

5. The fusion protein according to any one of claim 1 to 3, wherein the protein which includes a second tag is a LC3 protein mutant which includes the second tag and in which glycine at a carboxyl-terminus of the LC3 protein is deleted or substituted.

6. The fusion protein according to claim 5, wherein the fusion protein includes the second tag and the LC3 protein mutant in which glycine at a carboxyl-terminus of the LC3 protein is deleted or substituted in this order from the amino-terminus to the carboxyl-terminus.

7. A nucleic acid comprising
a base sequence coding for the fusion protein according to any one of claims 1 to 6.

8. A vector comprising
the nucleic acid according to claim 7.

9. A cell comprising
at least one of the nucleic acid according to claim 7 and the vector according to claim 8,
the at least one of the nucleic acid and the vector being introduced into the cell.

10. A transgenic non-human organism comprising
at least one of the nucleic acid according to claim 7 and the vector according to claim 8,
the at least one of the nucleic acid and the vector being introduced into the transgenic non-human organism.

11. A method for measuring autophagy, the method comprising
using at least one of the cell according to claim 9 and the transgenic non-human organism according to claim 10.
